# DEMANDE DE BREVET EUROPEEN

(11) **EP 4 044 190 A1**
(43) Date de publication de la demande: **17.08.2022**
(21) Numéro de dépôt: 21156213.7
(22) Date de dépôt: 10.02.2021
(51) Int. Cl.: G16H 10/20, G16H 15/00, G16H 50/20

(54) **SYSTÈME INTERACTIF DE SUPPORT À UNE DÉMARCHE D' ÉVALUATION VÉTÉRINAIRE**

(71) Demandeur: Ceva Santé Animale, 33500 Libourne (FR)
(72) Inventeur: LE TALLEC, Bertrand, 33500 LIBOURNE (FR); ROULLEAU, François, 33500 LIBOURNE (FR); LEORAT, Jean, 33500 LIBOURNE (FR)
(74) Mandataire: Aquinov

(57) **Abrégé**

L'invention concerne un système (1) interactif de support à une démarche d'évaluation vétérinaire comportant : un équipement connecté (2) apte à être porté sur la tête d'un vétérinaire et équipé d'un système d'acquisition et d'émission audio (23,24) et d'un système de communication sans-fil (27) ; un terminal mobile (3) équipé d'un écran (31), d'une interface (31) et d'un contrôleur (A) agencé pour afficher un questionnaire de consultation sur l'écran du terminal mobile et pour générer au moins un commentaire (Cp) relatif à une consultation réalisée par le vétérinaire en fonction d'au moins l'une des réponses (RI) apportées au questionnaire via l'interface.

## Description

L'invention concerne le domaine technique des activités vétérinaires. Plus précisément, l'invention a pour objet un système interactif de support à une démarche d'évaluation vétérinaire.

Dans le domaine des services vétérinaires, il est connu de faire appel à un vétérinaire pour organiser une visite de contrôle, d'évaluation ou de diagnostic dans un élevage industriel ou non, ou encore dans une exploitation agroalimentaire. C'est notamment le cas lorsque l'élevage ou l'exploitation est confronté à une problématique sanitaire ou zootechnique, et particulièrement en cas de suspicion de maladie chez les animaux. Par exemple, dans le cadre d'une exploitation avicole, cette visite peut permettre notamment de diagnostiquer l'apparition d'une maladie dans l'élevage comme une maladie de Newcastle (également nommée « ND », de l'anglais Newcastle Disease), de bronchite infectieuse (également nommée « IB » de l'anglais Infectious Bronchitis), de laryngotrachéite infectieuse (également nommée « ILT », de l'anglais Infectious LaryngoTracheitis), de maladie de Gumboro (également nommée « IBD », de l'anglais Infectious Bursal Disease) ou de grippe aviaire..

Toutefois, lors de l'organisation de la visite, le vétérinaire ne dispose que de peu d'informations quant à la ou les raisons de son intervention. Or, cette ou ces raisons peuvent être multiples et relever chacune d'un domaine ou d'une discipline spécifique comme, entre autres, la zootechnie, l'infectiologie, l'épidémiologie, la pathologie clinique, la biosécurité, la gestion de l'élevage, ou l'immunisation, voire d'une branche particulière de ces domaines et disciplines. Ceci implique que le vétérinaire, malgré son expérience multidisciplinaire, puisse avoir besoin d'une expertise spécifique afin de mener à bien son évaluation. C'est notamment le cas lorsque l'élevage ou l'exploitation fait appel à un vétérinaire généraliste ou à un vétérinaire en début de carrière. En outre, les informations dont il dispose avant la visite peuvent induire un biais dans son évaluation et l'empêcher de procéder à une analyse multifactorielle et pluridisciplinaire. Ces points peuvent être particulièrement critique dans un contexte d'élevage industriel, dans la mesure où une pathologie mal diagnostiquée ou diagnostiquée en retard peut causer une erreur ou un délai de traitement entrainant des dommages importants.

Une solution pourrait consister à organiser une visite conjointe du vétérinaire avec un expert spécialiste de l'espèce. Toutefois, il n'est pas toujours possible d'organiser une visite, dans un temps acceptable, d'un expert disposant de ces compétences requises, d'une part du fait du nombre croissant de consultations au regard du nombre d'experts disponibles et d'autre part du fait des coûts qu'un tel déplacement engendre.

Il existe ainsi un besoin pour une solution permettant à un vétérinaire de réaliser une consultation in situ dans une exploitation agricole, à des fins de contrôle, d'évaluation ou de diagnostic, et lui permettant de faire appel à une expertise spécifique afin que le contrôle, l'évaluation ou le diagnostic puisse être réalisé le plus rapidement possible et de façon la plus fiable possible.

La présente invention se place dans ce contexte et vise à répondre à ce besoin.

A ces fins, l'invention a pour objet un système interactif de support à une démarche d'évaluation vétérinaire comportant :
a. un équipement connecté apte à être porté sur la tête d'un vétérinaire et équipé d'un système d'acquisition et d'émission audio et d'un système de communication sans-fil ;
b. un terminal mobile équipé d'un écran, d'une interface et d'un contrôleur agencé pour afficher un questionnaire de consultation sur l'écran du terminal mobile et pour générer au moins un commentaire relatif à une consultation réalisée par le vétérinaire en fonction d'au moins l'une des réponses apportées au questionnaire via l'interface.

Grâce à l'invention, un vétérinaire équipé d'une part de l'équipement connecté et d'autre part du terminal mobile peut organiser une visite dans une exploitation agricole. L'équipement connecté lui permet de dialoguer avec un expert distant, lequel dispose des compétences spécifiques requises pour l'objet de son intervention. De la sorte, le vétérinaire peut être accompagné par cet expert pendant la consultation tout en ayant les mains libres pour réaliser les manipulations nécessaires à cette consultation ou pour interagir avec le terminal mobile. En outre, l'utilisation du terminal mobile et du questionnaire permet de suivre une démarche précise, notamment selon différents scénarii chacun adapté à une situation donnée, afin d'affiner l'évaluation ou de préciser des suspicions de maladie. Ce questionnaire, rempli par le vétérinaire et avec le support de l'expert distant, permet ainsi de guider et de faciliter la consultation, afin d'identifier des points clés lors de la visite. Il est à noter qu'à l'inverse d'une téléconsultation, le système selon l'invention permet au vétérinaire in situ de conserver son rôle au regard des exploitants et donc de maintenir une relation de confiance entre eux. A l'issue du questionnaire, le ou les commentaires générés par le contrôleur du terminal mobile permettent au vétérinaire de conclure la visite par un rapport de visite ou de contrôle ou par un diagnostic qu'il établit en fonction de ce ou ces commentaires. Bien évidemment, ce rapport pourra être un rapport préliminaire pouvant être enrichi, par le vétérinaire via le terminal mobile ou par l'expert à distance, par des données issues d'analyses d'échantillons réalisées a posteriori de la visite, par des informations supplémentaires, des compléments d'analyse ou des propositions de réponse ou de traitement. De même, une ou plusieurs visites complémentaires pourront également permettre d'enrichir, de façon itérative, ce rapport afin d'aboutir à un rapport final.

Selon l'invention, on entend par terminal mobile un ordinateur portable, un dispositif mobile, un téléphone intelligent ou une tablette électronique. Ce terminal mobile comprend divers composants matériels, dont un ou plusieurs processeurs à un ou plusieurs cœurs et une mémoire.

Le cas échéant, le contrôleur dudit terminal mobile pourra comporter tout ou partie desdits composants matériels, ainsi que des composants logiciels. De préférence, le contrôleur dudit terminal mobile pourra comporter une application logicielle, par exemple téléchargée dans une mémoire du terminal mobile et apte à contrôler les différents éléments du terminal mobile et notamment l'écran du terminal mobile, par exemple en fonction des données saisies par l'intermédiaire de l'interface. En variante, ladite application logicielle pourra être partiellement téléchargée dans une mémoire du terminal mobile et hébergée partiellement sur un serveur distant. Dans d'autres cas, l'application logicielle pourra être partiellement ou totalement virtualisé par le biais d'une architecture en nuage.

On entend par interface du terminal mobile une interface de saisie de données, par exemple une interface tactile, une interface vocale ou une interface gestuelle.

On entend également par équipement connecté apte à être porté sur la tête d'un vétérinaire un casque, un bandeau de tête, un accessoire de lunettes ou encore une paire de lunettes munie ou non de verres de protection. Le cas échéant, l'équipement connecté pourra être un équipement multimédia, notamment à réalité augmentée.

Avantageusement, le terminal mobile comporte une mémoire dans laquelle sont stockées une pluralité de questions prédéterminées, le questionnaire étant formé par une séquence de questions sélectionnées parmi ladite pluralité de questions et ordonnées selon un ordre déterminé. Le cas échéant, le contrôleur peut être agencé pour afficher sur l'écran du terminal mobile, de façon séquentielle selon ledit ordre déterminé, des blocs de questions comprenant au moins une question de ladite séquence ainsi que, pour chaque question dudit bloc, un composant graphique de réponse à ladite question. On entend par composant graphique de réponse un composant d'interface graphique avec lequel le vétérinaire peut interagir, via l'interface, pour répondre à une question, et notamment l'un des composants suivants ou encore une combinaison de plusieurs des éléments suivants : un bouton, une liste déroulante, une case à cocher, une zone de saisie de texte. En d'autres termes, le contrôleur peut être agencé, en réponse à une interaction du vétérinaire avec un composant graphique de réponse via l'interface, pour enregistrer dans ladite mémoire la réponse apportée à la question associée audit composant graphique de réponse via ce composant graphique de réponse.

Par exemple, ladite pluralité de questions prédéterminées peut être stockée dans ladite mémoire sous forme de groupes de questions prédéterminées. Si on le souhaite, chaque question peut être stockée dans la mémoire en étant associée à un index, chaque groupe étant formé par des questions de même index, lesdits blocs de questions étant formés à partir de questions appartenant à un même groupe. Le cas échéant, un même groupe peut fournir des questions à des blocs différents. En variante, lesdits blocs de questions peuvent être formés à partir de questions appartenant à différents groupes. Par exemple, chaque groupe de questions peut-être un groupe de question associé à un index indiquant l'un des sujets suivants : épidémiologie, paramètres zootechniques et techniques d'élevage, biosécurité, gestion, consultation, autopsie, prélèvement, immunisation, historique, analyse.

De préférence, le contrôleur peut être agencé pour afficher sur l'écran du terminal mobile un composant graphique de commande, et peut être agencé, en réponse à une interaction du vétérinaire avec ledit composant graphique de commande via l'interface, pour afficher le bloc de question suivant selon ledit ordre déterminé.

Avantageusement, le contrôleur peut être agencé pour afficher sur l'écran du terminal mobile une interface de sélection d'une porte d'entrée parmi une pluralité de portes d'entrée prédéterminées et pour sélectionner une séquence de questions parmi une pluralité de questions prédéterminées en fonction de la porte d'entrée sélectionné, ladite séquence de questions sélectionnée formant ledit questionnaire. En d'autres termes, pour deux portes d'entrée distinctes, les questions peuvent être différentes d'une séquence à l'autre et/ou être affichées dans un ordre différent d'une séquence à l'autre. Par exemple, chaque porte d'entrée peut être l'une des thématiques suivantes : suspicion d'échec d'une campagne de vaccination, épidémie respiratoire, diagnostic en laboratoire, enquête épidémiologique, sensibilisation aux maladies, évaluation d'un programme de vaccination, suivi, innovation. Chaque porte d'entrée pourra par exemple être suivie d'une porte d'entrée secondaire, notamment choisie parmi les sous-thématiques suivantes : suspicion d'une maladie de Newcastle, suspicion d'une bronchite infectieuse, suspicion d'une laryngotrachéite infectieuse, suspicion d'une maladie de Gumboro, suspicion d'une grippe aviaire.

En variante, le contrôleur pourra être agencé pour sélectionner une pluralité de questions parmi une pluralité de questions prédéterminées en fonction de la porte d'entrée sélectionnée, ladite séquence de questions sélectionnées formant un bloc de questions. Le cas échéant, le contrôleur pourra être agencé pour sélectionner une nouvelle pluralité de questions parmi une pluralité de questions prédéterminées en fonction des réponses apportées audit bloc de questions, ladite nouvelle séquence de questions sélectionnées formant un nouveau un bloc de questions, ces étapes étant renouvelées jusqu'à ce que le contrôleur sélectionne une pluralité de questions finales formant un bloc de question terminal, l'ensemble des blocs de questions successifs formant ledit questionnaire.

Dans un mode de réalisation de l'invention, pour au moins l'une, notamment chacune, desdites questions de ladite séquence, le contrôleur est agencé pour déterminer un score de réponse en fonction de la réponse apportée à ladite question, et pour générer ledit commentaire relatif à la consultation en fonction d'au moins l'un desdits scores déterminés. Par exemple, l'ensemble des réponses susceptibles d'être apportées à chaque question pourra être prédéfini et stocké dans ladite mémoire du terminal mobile, chacune de ces réponses étant stockée dans ladite mémoire en étant associée à un score prédéterminé. De façon alternative ou cumulative, chaque question de ladite séquence pourra faire l'objet d'une réponse quantifiable, de sorte qu'un score puisse être calculé par le contrôleur à partir de cette réponse, par exemple par comparaison de ladite réponse à un ou plusieurs seuils.

Avantageusement, une pluralité de commentaires prédéterminés sont stockés dans la mémoire du terminal mobile. Le cas échéant, le contrôleur pourra être agencé pour procéder à une comparaison d'au moins l'un desdits scores déterminés, ou encore à une comparaison d'une combinaison, notamment d'une somme, de plusieurs desdits scores déterminés, à une ou plusieurs valeurs seuils et pour sélectionner un commentaire parmi ladite pluralité de commentaires en fonction du résultat de ladite comparaison.

Avantageusement, pour chaque bloc de question, le contrôleur est agencé pour générer un commentaire relatif à la consultation en fonction d'au moins un score déterminée en fonction d'une réponse apportée à l'une des questions dudit bloc. En d'autres termes, le contrôleur générera au moins autant de commentaires que le questionnaire comporte de blocs.

Selon un exemple de réalisation de l'invention, pour au moins un bloc de questions, le contrôleur est agencé pour afficher une partie seulement des questions de ce bloc, dite préliminaire, et pour afficher le reste des questions de ce bloc en fonction d'au moins un score déterminée en fonction d'une réponse apportée à l'une des questions de ladite partie préliminaire. Il est ainsi possible de simplifier le remplissage du questionnaire par le vétérinaire, en évitant l'affichage de questions non pertinentes. Selon encore un exemple, pour au moins un bloc de questions, le contrôleur est agencé pour afficher une partie seulement des questions de ce bloc, en fonction d'au moins une réponse apportée à l'une des questions d'un bloc de questions précédent.

Avantageusement, à l'issue du remplissage du questionnaire par le vétérinaire, le contrôleur est agencé pour afficher, sur l'écran du terminal mobile, l'ensemble des commentaires relatifs à la consultation générés par le contrôleur à l'issue du remplissage du questionnaire, ainsi qu'un ensemble de composants graphiques de sélection chacun associé à l'une des commentaires affichés, le contrôleur étant agencé pour générer un rapport de consultation contenant les commentaires relatifs à la consultation, sélectionnés au moyen desdits composants graphiques de sélection. Par exemple, le contrôleur pourra être agencé pour autoriser la sélection d'un nombre prédéterminé de commentaires. Dans cet exemple, chaque commentaire prédéterminé stocké dans la mémoire du terminal mobile pourra être associé à une catégorie de commentaire choisie parmi une liste de catégories prédéterminées. Le cas échéant, le contrôleur pourra être agencé pour afficher lesdits commentaires générés de façon regroupée et ordonnée selon leurs catégories associées.

Dans un mode de réalisation de l'invention, le contrôleur dudit terminal mobile peut être agencé, en réponse à une interaction prédéterminée du vétérinaire avec l'interface, comme par exemple un appui sur un bouton, pour afficher sur l'écran du terminal mobile un composant graphique d'organisation d'une visite dans une exploitation agricole, notamment comportant un sous-composant graphique destiné à l'identification d'une exploitation agricole à visiter et/ou un sous-composant graphique de type agenda.

Dans un mode de réalisation de l'invention, le contrôleur dudit terminal mobile peut être agencé, en réponse à une interaction prédéterminée du vétérinaire avec l'interface, comme par exemple un appui sur un bouton, pour afficher sur l'écran du terminal mobile un composant graphique de réservation d'un expert distant. Le cas échéant, le contrôleur dudit terminal mobile peut être agencé pour envoyer un email de réservation audit expert distant identifié dans ledit composant graphique de réservation et/ou pour réserver un horaire dans un agenda dudit expert distant identifié dans ledit composant graphique de réservation.

Dans un mode de réalisation de l'invention, le contrôleur dudit terminal mobile peut être agencé, en réponse à une interaction prédéterminée du vétérinaire avec l'interface, comme par exemple un appui sur un bouton, pour afficher sur l'écran du terminal mobile un composant graphique de saisie de données relatives à un programme de vaccination d'animaux de ladite exploitation agricole.

Dans un mode de réalisation de l'invention, l'équipement connecté est une paire de lunettes comportant un module de communication sans-fil, un système d'acquisition vidéo et un écran, le système comportant une unité informatique distante équipée d'un système d'acquisition et d'émission audio, d'un écran et d'un module de communication sans-fil apte à communiquer avec le module de communication sans-fil de l'équipement connecté. Selon cette caractéristique, l'unité informatique distante est destinée à être utilisée par l'expert distant pour communiquer, oralement, avec le vétérinaire et pour visualiser, au moyen de l'écran de l'unité informatique distante, la visite du vétérinaire et ses différentes actions, comme par exemple une autopsie.

Le cas échéant, le module de communication sans-fil de la paire de lunettes peut être agencé pour transmettre au module de communication sans-fil de l'unité informatique distante des signaux audio et/ou vidéo acquis par le système d'acquisition audio et/ou le système d'acquisition vidéo de l'équipement connecté ou pour recevoir du module de communication sans-fil de l'unité informatique distante des signaux audio acquis par le système d'acquisition audio de l'unité information distante. Par exemple, le module de communication sans-fil de la paire de lunettes peut être agencé pour communiquer avec un réseau de télécommunication, notamment un réseau de télécommunications de type LTE, par exemple de 4^{ème} ou de 5^{ème} génération, de type Wifi ou encore de type Bluetooth. De préférence, le système d'acquisition vidéo de la paire de lunettes est une caméra.

Avantageusement, le module de communication sans-fil de la paire de lunettes est agencé pour transmettre les images acquises par le système d'acquisition vidéo de la paire de lunettes au module de communication sans-fil de l'unité informatique, l'unité informatique étant agencée pour afficher sur l'écran de l'unité informatique les images reçues. Le cas échéant, l'unité informatique comporte une interface de modification d'au moins l'une desdites images reçues, et l'unité informatique est agencée pour transmettre au module de communication sans-fil de de la paire de lunettes, via son module de communication sans-fil, une image modifiée au moyen de ladite interface de modification, la paire de lunettes étant agencée pour afficher ladite image modifiée sur l'écran de la paire de lunettes. Selon cette caractéristique, il est possible à l'expert de faire une capture d'écran dans la retransmission visuelle de la visite du vétérinaire sur l'écran de l'unité informatique, par exemple au cours d'une autopsie ou d'une observation d'une zone de l'exploitation agricole, puis de modifier l'image obtenue via l'interface de modification, et de retransmettre cette image modifiée au vétérinaire via l'écran de la paire de lunettes afin de lui montrer un point d'intérêt. On entend notamment par modification d'une image l'une des modifications suivantes ou encore une combinaison de plusieurs des modifications suivantes : grossissement, ajout d'une flèche, d'un cercle ou de texte à l'image.

L'invention a également pour objet un procédé de support à une démarche d'évaluation vétérinaire mis en œuvre par un terminal mobile d'un système de consultation selon l'invention.

Dans un mode de réalisation, le procédé comporte les étapes suivantes :
a. Sélection dans une mémoire du terminal mobile d'une séquence de questions parmi une pluralité de questions prédéterminées ;
b. Affichage séquentiel sur l'écran du terminal mobile de blocs de questions comportant chacun au moins une question de ladite séquence et pour chaque question de chaque bloc, un composant graphique de réponse à ladite question ;
c. Génération d'au moins un commentaire relatif à ladite consultation en fonction d'au moins l'une des réponses apportées aux questions de ladite séquence de question via l'interface du terminal mobile.

De préférence, ladite séquence de questions sélectionnées est ordonnée selon un ordre déterminé, au préalable ou dynamiquement. Pour chaque bloc de questions, l'étape d'affichage pourra comporter l'affichage d'un composant graphique de commande, de sorte qu'une interaction, via l'interface, avec ce composant graphique de commande entraine l'affichage, à la place du bloc de questions courant, du bloc de questions suivant, c'est à-dire le bloc de questions comportant les questions de la séquence de questions succédant aux questions du bloc de questions courant selon ledit ordre déterminé.

Avantageusement, le procédé comporte une étape préalable d'affichage, sur l'écran du terminal mobile, d'une interface de sélection d'une porte d'entrée parmi une pluralité de portes d'entrée prédéterminées stockées dans ladite mémoire, ladite séquence de questions étant sélectionnées en fonction de ladite porte d'entrée sélectionnée via l'interface.

Avantageusement, le procédé comporte, pour chaque bloc de questions affiché sur l'écran du terminal mobile, une étape de détermination d'au moins un score de réponse à l'une des questions dudit bloc de questions en fonction de la réponse apportée à ladite question à l'aide de l'interface. Le cas échéant, ledit au moins un commentaire relatif à la consultation est généré en fonction d'au moins l'un des scores de réponses déterminé. De préférence, l'étape de génération comporte, pour chaque bloc de questions, la génération d'au moins un commentaire relatif à la consultation, en fonction d'au moins l'un des scores de réponses déterminé à partir de la réponse apportée à l'une des questions de ce bloc de questions.

Avantageusement, pour au moins une question d'au moins un des blocs de questions, notamment chacun des blocs de questions, le composant graphique de réponse à ladite question affiché sur l'écran du terminal mobile est un composant de sélection, via l'interface, d'une réponse parmi une pluralité de réponses prédéterminées à ladite question, lesdites réponses étant stockées dans ladite mémoire du terminal mobile en étant associée à un score prédéterminé. Le cas échéant, le score de réponse déterminé à partir de la réponse apportée à ladite question est le score associé à la réponse sélectionnée via l'interface. Ledit composant de sélection pourra par exemple être une pluralité de boutons indiquant chacun l'une des réponses prédéterminées ou en variante une liste déroulante dont chaque item est l'une des réponses prédéterminées.

De façon alternative ou cumulative, pour au moins une question d'au moins un des blocs de questions, notamment chacun des blocs de questions, le composant graphique de réponse à ladite question affiché sur l'écran du terminal mobile est un composant de saisie d'une réponse quantifiable, par exemple un nombre, via l'interface. Le cas échéant, le score de réponse déterminé à partir de la réponse apportée à ladite question est calculé en fonction de la réponse saisie via l'interface, par exemple par comparaison dudit nombre à un ou plusieurs seuils.

De préférence, une pluralité de commentaires prédéterminés sont stockés dans ladite mémoire du terminal mobile. Le cas échéant, l'étape de génération dudit au moins un commentaire relatif à la consultation, en fonction d'au moins une réponse apportée à l'une des questions de ladite séquence de question, comporte la comparaison du score de réponse déterminé à partir de ladite réponse à une ou plusieurs valeurs seuils, et la sélection d'un commentaire parmi ladite pluralité de commentaires prédéterminés en fonction du résultat de ladite comparaison. Eventuellement, ledit commentaire pourra être sélectionné en fonction du résultat d'une comparaison d'une combinaison, notamment d'une somme, de plusieurs scores déterminés à partir des réponses apportées à différentes questions d'un même bloc de questions, à une ou plusieurs valeurs seuils. De préférence, chaque commentaire prédéterminé d'un groupe de commentaires pourra être associé, dans ladite mémoire du terminal, à un résultat prédéterminé d'une comparaison.

Avantageusement, pour au moins l'un des blocs de questions, l'étape d'affichage pourra comprendre l'affichage d'une partie seulement des questions de ce bloc, dite partie préliminaire, la détermination d'au moins un score de réponse à l'une des questions de ladite partie préliminaire en fonction de la réponse apportée à ladite question à l'aide de l'interface, et, en fonction d'une comparaison dudit score de réponse à une ou plusieurs valeurs seuils, l'affichage du reste des questions de ce bloc.

Avantageusement, à l'issue de l'étape de génération d'au moins un commentaire relatif à ladite consultation, le procédé comporte une étape d'affichage, sur l'écran du terminal mobile, de l'ensemble des commentaires relatifs à la consultation générés, ainsi qu'un ensemble de composants graphiques de sélection chacun associé à l'une des commentaires affichés. Le cas échéant, le procédé peut comporter une étape ultérieure de génération d'un rapport de consultation contenant les commentaires relatifs à la consultation sélectionnés au moyen desdits composants graphiques de sélection via l'interface.

L'invention a également pour objet un programme d'ordinateur comprenant un code de programme qui est conçu pour mettre en œuvre le procédé selon l'invention lorsque ledit programme est exécuté par un ordinateur.

La présente invention est maintenant décrite à l'aide d'exemples uniquement illustratifs et nullement limitatifs de la portée de l'invention, et à partir des dessins annexés, dessins sur lesquels les différentes figures représentent :
[Fig. 1] représente, partiellement et schématiquement, un système de consultation vétérinaire selon un exemple de réalisation de l'invention ;
[Fig. 2] représente, partiellement et schématiquement, une image acquise par la caméra de la paire de lunettes du système de consultation de la [Fig. 1], modifiée par l'expert distant et transmise à l'écran de la paire de lunettes, lors d'une autre étape du procédé de la [Fig. 2];
[Fig. 3] représente, partiellement et schématiquement, un procédé de support à une consultation mis en œuvre par le terminal mobile du système de consultation de la [Fig. 1] ; et
[Fig. 4] représente, partiellement et schématiquement, une vue de l'écran du terminal mobile du système de consultation de la [Fig. 1] lors d'une étape du procédé de la [Fig. 3] ;
[Fig. 5] représente, partiellement et schématiquement, une vue de l'écran du terminal mobile du système de consultation de la [Fig. 1] lors d'une autre étape du procédé de la [Fig. 3] ;
[Fig. 6] représente, partiellement et schématiquement, une vue de l'écran du terminal mobile du système de consultation de la [Fig. 1] lors d'une autre étape du procédé de la [Fig. 3] ;
[Fig. 7] représente, partiellement et schématiquement, une vue de l'écran du terminal mobile du système de consultation de la [Fig. 1] lors d'une autre étape du procédé de la [Fig. 3];
[Fig. 8] représente, partiellement et schématiquement, une vue de l'écran du terminal mobile du système de consultation de la [Fig. 1] lors d'une autre étape du procédé de la [Fig. 3] ; et
[Fig. 9] représente, partiellement et schématiquement, une vue de l'écran du terminal mobile du système de consultation de la [Fig. 1] lors d'une autre étape du procédé de la [Fig. 3] .

Dans la description qui suit, les éléments identiques, par structure ou par fonction, apparaissant sur différentes figures conservent, sauf précision contraire, les mêmes références.

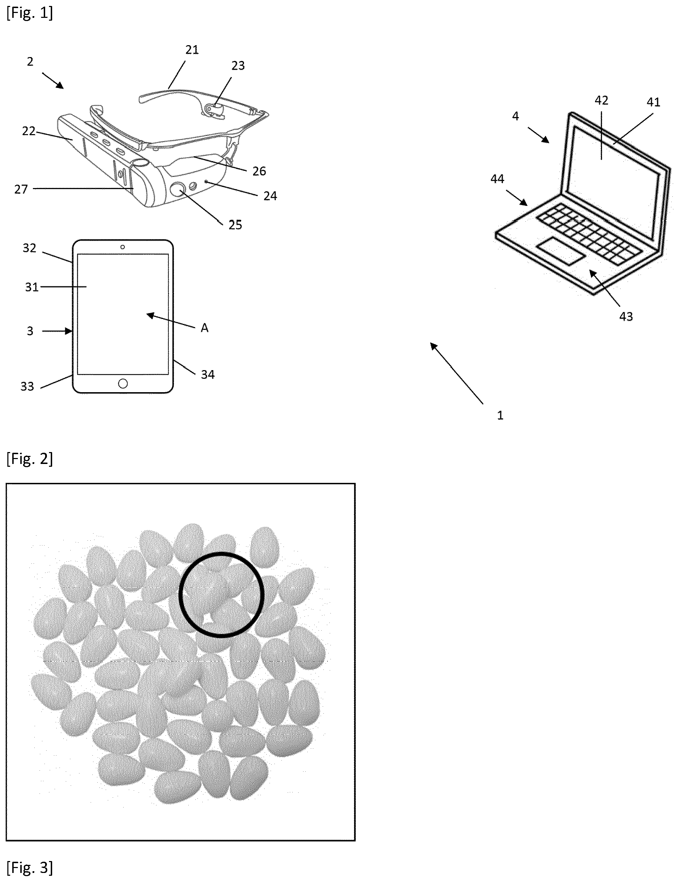

On a décrit en [Fig. 1] un système de consultation vétérinaire 1, notamment destiné à une consultation d'une exploitation avicole ou d'un couvoir ou encore d'un abattoir. Dans l'exemple qui va être décrit, le système 1 est destiné à être utilisé, d'une part, par un vétérinaire visitant l'exploitation avicole ou le couvoir, à des fins de diagnostic, d'évaluation ou de contrôle, et d'autre part, par un expert distant du vétérinaire, disposant d'un ensemble de compétences spécifiques aux volatiles de cette exploitation avicoles, comme par exemple des compétences relatives au diagnostic de maladies susceptibles de les contaminer et aux traitements ou vaccins permettant de traiter ces maladies.

Le système 1 comporte un équipement connecté 2, représenté dans cet exemple sous la forme d'une paire de lunettes 2 équipée de branches 21 pour permettre son maintien sur la tête du vétérinaire. Dans l'exemple décrit, la paire de lunettes 2 est dépourvue de verres de protection, mais dispose d'un pont situé entre les branches et destiné à reposer sur le nez du vétérinaire, l'espace entre le pont et les branches étant apte à recevoir des verres de protection. En variante, on pourrait envisager de remplacer les branches 21 par un bandeau ou tout autre moyen de maintien de l'équipement connecté 2 sur la tête du vétérinaire.

La paire de lunettes 2 comporte un module 22 monté sur l'une des branches 21. La paire de lunettes 2 comporte un système d'acquisition et d'émission audio comprenant un haut-parleur 23, disposé sur la branche 21 opposée à celle recevant le module 22 et destiné à venir en vis-à-vis de l'oreille du vétérinaire, et un microphone 24, prévu sur une aile du module 22. La paire de lunettes 2 comporte une caméra 25, disposée sur l'aile du module 22, au droit du microphone 24, ainsi qu'un écran 26, disposé sur la face opposée de l'aile du module 22 recevant la caméra 25 et le microphone 24 de sorte à venir en vis-à-vis de l'œil du vétérinaire.

Le module 22 embarque en outre un module de communication sans-fil 27, apte à émettre des trames de données selon un protocole Wifi et/ou un protocole Bluetooth, ainsi qu'une batterie (non représentée) destinée à alimenter l'ensemble des éléments électroniques de la paire de lunettes 2 et une pluralité de boutons et/ou d'interfaces tactiles destinés à contrôler ces différents éléments électroniques.

Le système 1 comporte également un terminal mobile 3, représenté dans cet exemple sous la forme d'une tablette tactile 3, destiné à équiper le vétérinaire portant la paire de lunettes 2.

La tablette tactile 3 est équipée d'un écran tactile 31, formant à la fois un écran et une interface de saisie, d'un ensemble de composants électroniques 32 comprenant notamment un processeur, ainsi que d'une mémoire 33. Une application logicielle A est stockée dans la mémoire 33 et est apte à contrôler l'affichage de différents composants graphiques sur l'écran tactile 31, notamment en fonction d'interactions du vétérinaire avec l'écran tactile 31, comme des appuis ponctuels ou continus, des glissements de doigts ou des saisies de données. L'application A fait ainsi partie d'un contrôleur de la tablette tactile 3.

La tablette tactile 3 comporte également un module de communication sans-fil 34, apte à émettre des trames de données selon un protocole LTE et/ou un protocole Wifi et/ou un protocole Bluetooth, ainsi qu'une batterie (non représentée) destinée à alimenter l'ensemble des éléments électroniques de la tablette tactile 3.

Le système 1 comporte également une unité informatique distante 4, destinée à être manipulée par l'expert distant du vétérinaire, représentée dans cet exemple sous la forme d'un ordinateur portable 4.

L'ordinateur portable 4 comporte un système d'acquisition et d'émission audio 41, sous la forme d'un microphone et d'un haut-parleur, d'un écran 42, d'une interface 43, sous la forme d'un clavier et d'un pavé tactile, et d'un module de communication sans-fil 44, apte à émettre des trames de données selon un protocole LTE et/ou un protocole Wifi et/ou un protocole Bluetooth

Dans l'exemple décrit, la paire de lunettes 2 est connectée, par liaison Bluetooth, avec la tablette tactile 3. La paire de lunettes 2 peut ainsi échanger des signaux audio et vidéo avec l'ordinateur portable 4, soit directement par liaison Wifi via leurs modules de communication 27 et 44, soit via la tablette tactile 3, les signaux étant échangés entre la paire de lunettes 2 et la tablette tactile 3 par liaison Bluetooth et entre la tablette tactile 3 et l'ordinateur portable 4 par liaison LTE ou Wifi. De la sorte, le vétérinaire et l'expert distant peuvent communiquer oralement, via les système d'acquisition et d'émission audio 23/24 et 41. Par ailleurs, l'expert distant peut suivre, sur l'écran 42, la visite du vétérinaire, les images acquises par la caméra 25 de la paire de lunettes y étant retransmises. En outre, l'expert distant peut ainsi réaliser une capture d'écran dans cette retransmission, modifier l'image capturée au moyen de l'interface 43, par exemple en y insérant un cercle ou un flèche comme montré en [Fig. 2], puis transmettre cette image modifiée à la paire de lunettes 2 pour qu'elle soit affichée sur l'écran 26.

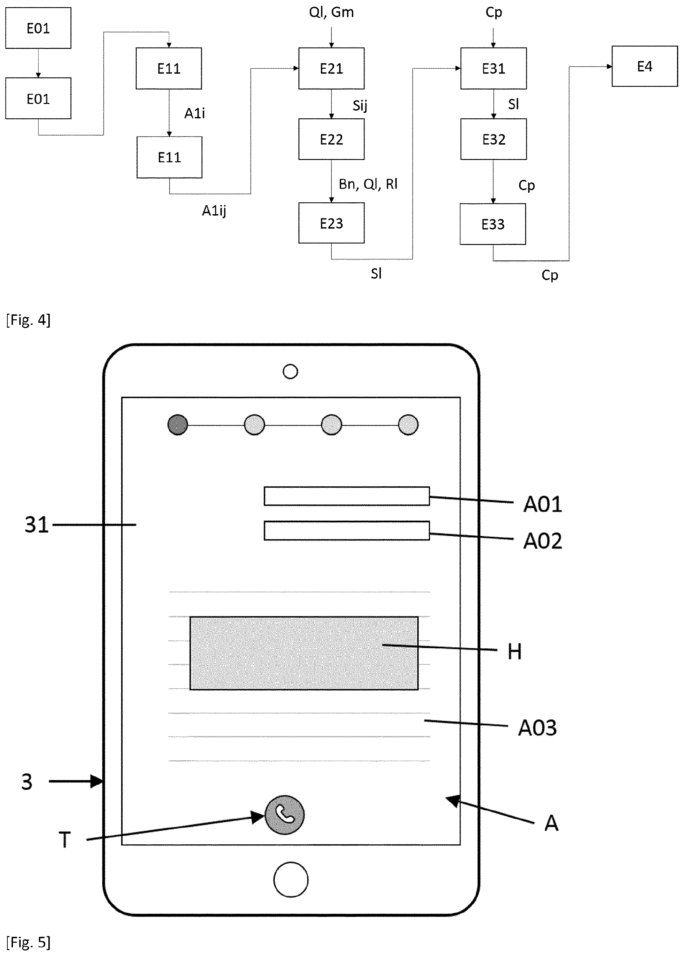

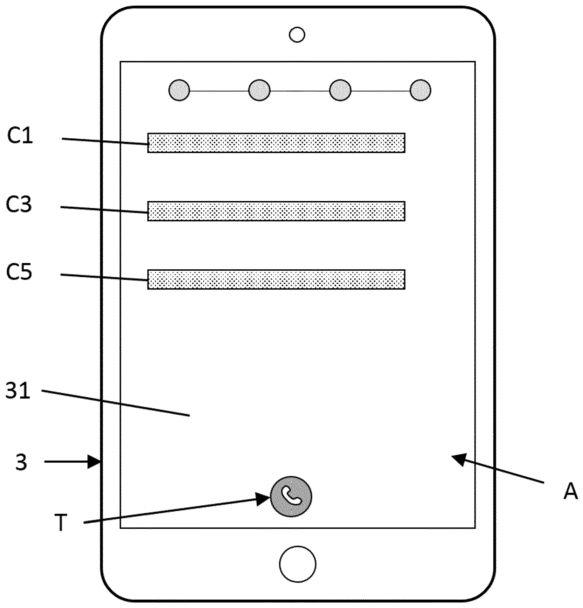

On va désormais décrire en [Fig. 3] un exemple de procédé mis en œuvre par l'application logicielle A lors de la consultation réalisée par le vétérinaire dans l'exploitation avicole ou le couvoir qu'il visite, l'application logicielle contrôlant l'écran 31 de la tablette 3, notamment en fonction des interactions du vétérinaire avec la tablette. La [Fig. 2] sera par la suite décrite en liaison avec les [Fig. 4] à [Fig. 9] qui montrent l'écran 31 de la tablette tactile 3 à différentes étapes du procédé de la [Fig. 3].

Au préalable de la visite, le vétérinaire peut accéder à une feuille de l'application A, dit gestionnaire de visite, permettant d'organiser ses visites. Dans une étape E01, à la suite d'une interaction du vétérinaire, via l'écran 31, avec un composant graphique de commande affiché sur l'écran 31 et dédié à ce gestionnaire de visite (non représenté), l'application A contrôle l'écran 31 pour y afficher ledit gestionnaire de visite. Le cas échéant, ledit gestionnaire pourra comporter une liste de toutes les visites prévues ou réalisées par le vétérinaire, ainsi qu'un composant graphique de création d'une nouvelle visite.

Dans une étape E02, à la suite d'une interaction du vétérinaire, via l'écran 31, avec ce composant graphique de création d'une nouvelle visite, l'application A contrôle l'écran 31 pour y afficher une page de création d'une nouvelle visite, représentée en [Fig. 4]. Cette page comporte une pluralité de composants graphiques, dont au moins un composant graphique A01 destiné à l'identification d'une exploitation avicole à visiter, un composant graphique de réservation d'un expert A02, et un composant graphique de type agenda A03. Dans l'exemple décrit, le composant A01 est une zone de saisie de texte et le composant A03 est un agenda dans lequel il est possible de réserver un créneau horaire.

Le composant graphique de réservation d'un expert A02 peut être une liste déroulante dont chaque item indique le nom d'un expert disponible, par exemple choisi parmi une liste d'experts prédéterminés. L'application A pourra par exemple obtenir ladite liste d'experts par interrogation d'une base de données distante, en fonction d'un horaire de visite H saisi par le vétérinaire, via le composant 103. Les composants A01 à 103 ont été représentés en [Fig. 3] en étant affichés sur une même page, étant entendu qu'ils pourront être affichés sur différents onglets d'une même page, et que cette page pourra comporter d'autres composants non représentés en [Fig. 3], notamment des composants d'identification de l'adresse de l'exploitation avicole, d'identification du nom de l'exploitant, et de saisie de données relatives à un programme de vaccination des volailles de cette exploitation avicole.

A noter qu'un composant graphique T, sous la forme d'un bouton, permet de déclencher un appel entre le vétérinaire et l'expert. Par exemple, à la suite d'une interaction du vétérinaire avec ce composant, l'application A transmet une instruction d'appel à destination de la paire de lunettes 2, la paire de lunettes 2 étant agencée pour, à la réception de cette instruction d'appel, initier un échange de signaux audio et/ou vidéo entre ladite paire de lunettes 2 et l'ordinateur portable 4.

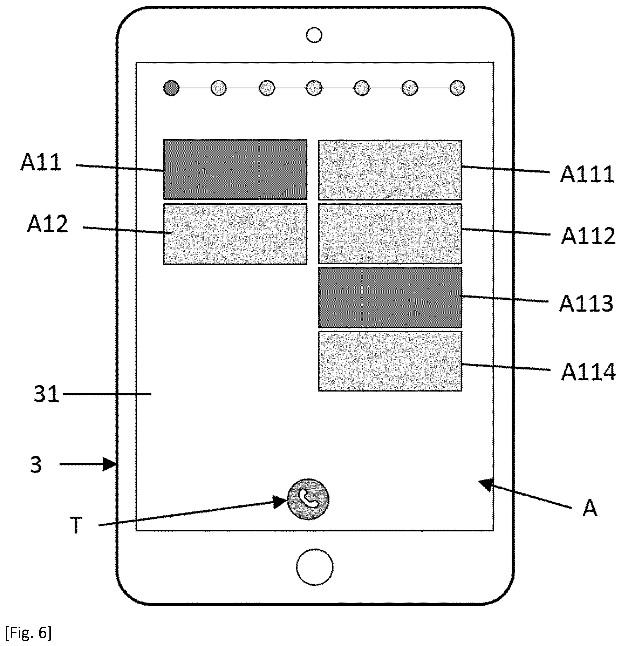

Au début de sa visite dans l'exploitation avicole, le vétérinaire peut initialiser l'affichage d'un questionnaire. Dans une étape E11, à la suite d'une interaction du vétérinaire, via l'écran 31, avec un composant graphique de commande affiché sur l'écran 31 et dédié à ce questionnaire (non représenté), l'application A contrôle l'écran 31 pour y afficher une interface de sélection d'une porte d'entrée parmi une pluralité de portes d'entrée prédéterminées, représentée en [Fig. 5].

Cette interface comporte une pluralité de composants graphiques de sélection Ali, en l'occurrence des boutons A11 et A12, indiquant chacun une thématique comme par exemple « suspicion d'échec d'une campagne de vaccination » et « épidémie respiratoire ». Chacune de ces thématiques est stockée dans la mémoire 33 de la tablette 3 et est ainsi affichée sur l'écran 31, sous la forme d'un composant graphique de sélection A11 ou A12, par l'application A.

Dans une étape E12, à la suite d'une interaction du vétérinaire, via l'écran 31, avec l'un des composants graphiques de sélection Ali, l'application A contrôle l'écran 31 pour y afficher une pluralité de composants graphiques de sélection supplémentaires Alij, en l'occurrence des boutons A111 à A114, définissant chacune une porte d'entrée prédéterminée, comme par exemple l'une des sous-thématiques suivantes : suspicion d'une maladie de Newcastle, suspicion d'une bronchite infectieuse, suspicion d'une laryngotrachéite infectieuse, suspicion d'une maladie de Gumboro. Une pluralité de sous-thématiques est stockée dans la mémoire 33 de la tablette 3, chaque sous-thématique étant associée à l'une des thématique stockée dans cette mémoire 33. Dès lors, la sélection par le vétérinaire de l'une des thématiques, au moyen des boutons Ali, permet à l'application A d'identifier, dans la mémoire 33, les sous-thématiques associées à la thématique sélectionnée et de les afficher, sur l'écran 31, sous la forme de composants graphiques de sélection Alij, de sorte que le vétérinaire puisse sélectionner l'une de ces sous-thématiques comme porte d'entrée du questionnaire.

Une pluralité de questions prédéterminées Ql est stockée dans la mémoire 33 de la tablette tactile 3, les questions Ql étant associées à un index m de sorte à pouvoir être regroupées en groupe de question Gm d'un même index m. Les questions Ql d'un même groupes Gm pourront par exemple être des questions portant sur un même sujet, comme par exemple l'épidémiologie, la gestion de l'exploitation avicole, l'historique de l'exploitation avicole, le programme de vaccination suivi, la biosécurité, la consultation ou l'autopsie.

A la suite de la sélection par le vétérinaire, via l'écran 31, d'une portée d'entrée Alij, l'application A sélectionne, dans une étape E21, dans la mémoire 33 et parmi les question Ql une séquence Sij de questions, définie au préalable et ordonnée selon un ordre déterminée. Il est à noter que la séquence Sij sélectionnée à partir de l'une des portée d'entrée Alij sera différente de la séquence Sik sélectionnée à partir d'une autre portée d'entrée Alik, les questions de la séquence Sij pouvant être différentes de celles de la séquence Sik, et/ou être identiques à celles de la séquence Sik mais ordonnées selon un ordre différent. Dans l'exemple décrit, le début de la séquence S13 qui a été sélectionnée, à la suite de la sélection de la porte d'entrée A113 par le vétérinaire, est formée des questions Ql à Q4 du groupe Gl, des questions Q5 et Q6 du groupe G2 et de la question G7 du groupe G3.

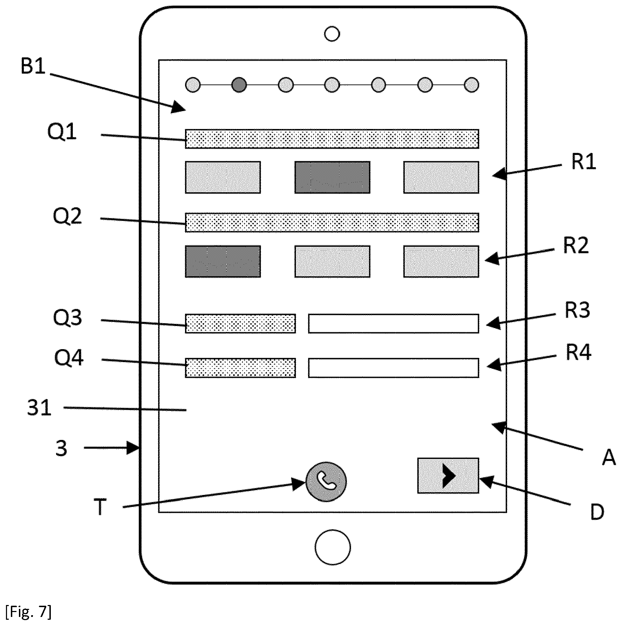

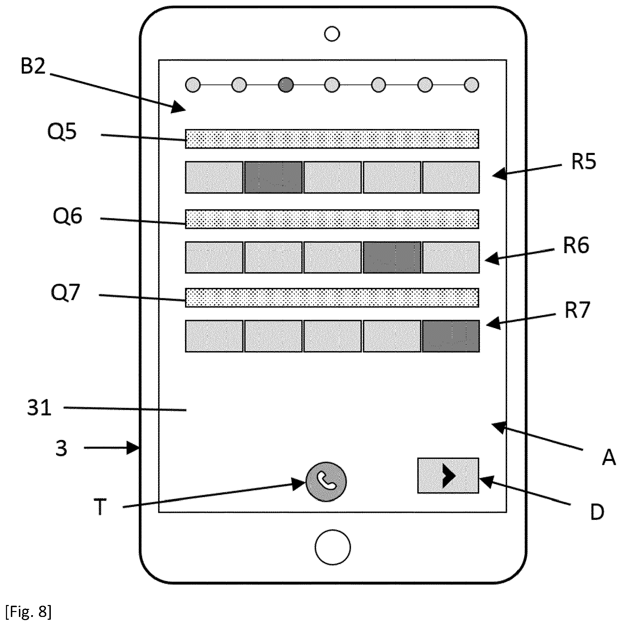

Dans une étape E22, l'application A affiche, sur l'écran 31, les questions Ql de la séquence sélectionnée Sij en les regroupant par blocs de question Bn affichés de façon séquentielle selon l'ordre de la séquence sélectionnée. Les [Fig. 6] et [Fig. 7] montrent chacune l'affichage du bloc Bl, respectivement B2, sur l'écran 31.

Dans l'exemple décrit, chaque bloc de questions Bn peut être formé par des questions Ql, voire toutes les questions, d'un même groupe de questions Gm, comme dans le cas du bloc B1 formé des questions Q1 à Q4 du groupe Gl, ou par des questions provenant de différents groupes, comme dans le cas du bloc B2, formé des questions Q5 et Q6 du groupe G2 et de la question G7 du groupe G3.

Pour chaque bloc Bn, l'application A affiche ainsi sur l'écran 31 l'ensemble des questions Ql de ce bloc Bn, ainsi que pour chaque question Ql de ce bloc Bn, un ou plusieurs composants graphiques de réponse Rl à cette question. Le type de composant graphique de réponse à une question donnée est prédéterminé et associé à cette question. Par exemple, pour une question Ql donnée, il peut être prévu plusieurs réponses possibles. Chacune de ces réponses possibles peut ainsi être affichée par l'application A sur l'écran 31, sous la forme d'un bouton, l'ensemble de ces boutons formant le composant graphique de réponse, comme pour les questions Ql, Q2 et Q5 à Q7. Selon un autre exemple, le composant graphique de réponse peut être une zone de saisie d'un nombre, comme dans le cas des questions Q3 et Q4.

Dans l'exemple décrit, un bloc seul Bn peut être affiché chacun sur la page de l'application A et il est prévu un composant graphique de commande D permettant de faire défiler les blocs Bn dans l'ordre déterminé de la séquence de questions. En d'autres termes, les questions et les blocs sont organisés de façon logique en fonction du but recherché par la consultation, ce but étant identifié au moyen de la porte d'entrée. Dans l'exemple décrit, si la porte d'entrée sélectionnée est une suspicion d'une maladie de Newcastle, les questions du bloc B1 peuvent avoir pour but la récolte de données épidémiologiques et les questions du bloc B2 peuvent avoir pour but la récolte de données d'analyses biologiques, tandis que les questions des blocs suivants peuvent avoir pour but la récolte de données environnementales, de données cliniques, de données relatives à la gestion de l'exploitation avicole, etc. On vient ainsi guider le vétérinaire dans sa consultation, grâce au questionnaire et avec le support de l'expert distant, si nécessaire.

Pour chaque question Ql, la réponse apportée par le vétérinaire, via le composant graphique de réponse Rl, est enregistrée par l'application A dans la mémoire 33 de la tablette 3.

Dans une étape E23, à l'issue du remplissage du questionnaire, indiqué par exemple par une interaction du vétérinaire avec un composant graphique de contrôle dédié, l'application A détermine, pour chaque bloc de question Bn, un score de réponse Sl à au moins l'une des questions Ql de ce bloc Bn, en fonction de la réponse apportée par le vétérinaire à cette question via le composant graphique de réponse Rl.

Par exemple, lorsque le composant graphique de réponse Rl permet au vétérinaire de sélectionner une réponse parmi plusieurs réponses possibles, chaque réponse possible peut être stockée dans la mémoire 33 de la tablette mobile en étant associée à un score donné. La sélection d'une réponse permet ainsi à l'application A d'attribuer à cette réponse sélectionnée le score qui lui est associé. Lorsque le composant graphique de réponse Rl permet au vétérinaire de saisir un nombre, ce nombre peut être comparé à un ou plusieurs seuils, le résultat de cette comparaison permettant à l'application A d'attribuer un score à la réponse.

Une pluralité de commentaires de consultation Cp sont stockés dans la mémoire 33 de la tablette mobile 3. Dans une étape E31, l'application A compare l'un des scores Sl, plusieurs des scores Sl, voir chaque score Sl déterminé à l'issue de l'étape E23, ou encore une ou plusieurs combinaisons de ces scores Sl, par exemple des sommes de ces scores Sl, à une ou plusieurs valeurs seuils prédéterminées.

Dans une étape E32, l'application A sélectionne au moins l'un des commentaires de consultations Cp en fonction du résultat de l'une de ces comparaisons ou d'une combinaison logique de plusieurs comparaisons. Par exemple, plusieurs commentaires de consultations Cp peuvent être stockés dans la mémoire 33 de la tablette tactile 3 en étant chacun associé à un résultat différent d'une même comparaison d'un score de réponse à une ou plusieurs valeurs seuils données. Dans l'exemple décrit, l'application A procède à une comparaison des scores de réponse S1, S2 et S7, déterminés respectivement à partir des réponses R1, R2 et R7, à des valeurs seuils donnés pour sélectionner les commentaires Cl, C2 et C5, tandis qu'elle procède à une comparaison de la somme des scores de réponse S3 et S4, déterminés respectivement à partir des réponses R3 et R4, à des valeurs seuils donnés pour sélectionner le commentaire C3 et à une comparaison de la somme des scores de réponse S5 et S6, déterminés respectivement à partir des réponses R5 et R6, à des valeurs seuils donnés pour sélectionner le commentaire C4.

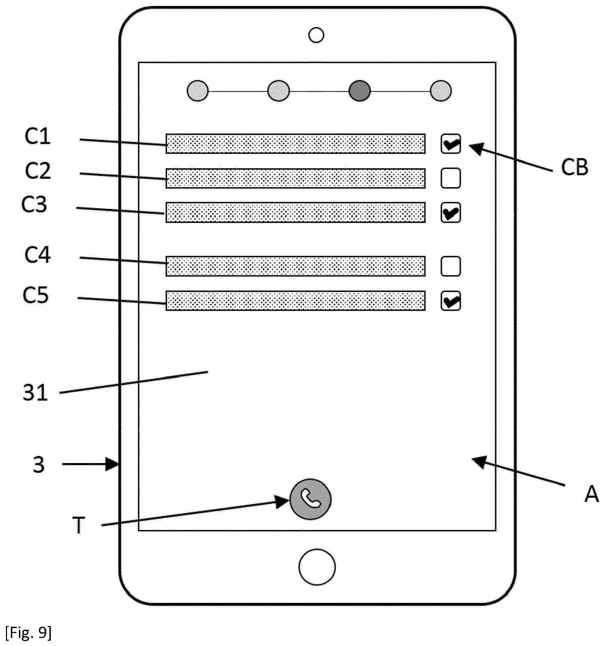

Dans une étape E33, l'application A affiche sur l'écran 31, les commentaires Cp générés à l'issue de l'étape E32, en les regroupant selon le bloc de question Bn ayant permis leur sélection, comme représenté en [Fig. 8].

L'application A affiche également, pour chaque commentaire Cp affiché, un composant graphique de sélection CB permettant au vétérinaire de sélectionner ou non ce commentaire.

Dans une étape E4, à l'issue de la sélection des commentaires par le vétérinaire, indiqué par exemple par une interaction du vétérinaire avec un composant graphique de contrôle dédié, l'application A génère un rapport de consultation contenant ces commentaires sélectionnés, comme montré en [Fig. 9], ainsi que l'ensemble des réponses apportées aux questions du questionnaire, lequel servira au vétérinaire pour justifier son diagnostic ou ses préconisations. Ce rapport pourra être un rapport préliminaire pouvant être enrichi, par le vétérinaire via le terminal mobile ou par l'expert à distance, par des données issues d'analyses d'échantillons réalisées a posteriori de la visite, par des informations supplémentaires, des compléments d'analyse ou des propositions de réponse ou de traitement. De même, une ou plusieurs visites complémentaires pourront également permettre d'enrichir, de façon itérative, ce rapport afin d'aboutir à un rapport final.

La description qui précède explique clairement comment l'invention permet d'atteindre les objectifs qu'elle s'est fixée, à savoir permettre à un vétérinaire de réaliser une consultation in situ dans une exploitation agricole et de contrôler, d'évaluer ou de diagnostiquer de la façon la plus rapide et la plus fiable possible, , grâce à la combinaison d'un équipement connecté lui permettant de dialoguer avec un expert distant, qui lui apportera, en sus de son expertise, l'assurance d'une démarche holistique et pertinente, et d'un terminal mobile muni d'un questionnaire permettant de guider et de faciliter la consultation, afin d'identifier des points clés lors de la visite.

En tout état de cause, l'invention ne saurait se limiter aux modes de réalisation spécifiquement décrits dans ce document, et s'étend en particulier à tous moyens équivalents et à toute combinaison techniquement opérante de ces moyens.

## Revendications

1. Système (1) interactif de support à une démarche d'évaluation vétérinaire comportant :
a. un équipement connecté (2) apte à être porté sur la tête d'un vétérinaire et équipé d'un système d'acquisition et d'émission audio (23,24) et d'un système de communication sans-fil (27) ;
b. un terminal mobile (3) équipé d'un écran (31), d'une interface (31) et d'un contrôleur (A) agencé pour afficher un questionnaire de consultation sur l'écran du terminal mobile et pour générer au moins un commentaire (Cp) relatif à une consultation réalisée par le vétérinaire en fonction d'au moins l'une des réponses (Rl) apportées au questionnaire via l'interface.

2. Système (1) selon la revendication précédente, dans lequel le terminal mobile (3) comporte une mémoire (33) dans laquelle sont stockées une pluralité de questions prédéterminées (Ql), le questionnaire étant formé par une séquence (Sij) de questions sélectionnées parmi ladite pluralité de questions et ordonnées selon un ordre déterminé, le contrôleur (A) étant agencé pour afficher sur l'écran (31) du terminal mobile, de façon séquentielle selon ledit ordre déterminé, des blocs de questions (Bn) comprenant au moins une question de ladite séquence ainsi que, pour chaque question dudit bloc, un composant graphique de réponse (Rl) à ladite question.

3. Système (1) selon la revendication précédente, dans lequel le contrôleur (A) est agencé pour afficher sur l'écran (31) du terminal mobile (3) une interface de sélection d'une porte d'entrée parmi une pluralité de portes d'entrée prédéterminées (Ali, Alij) et pour sélectionner une séquence de questions (Sij) parmi une pluralité de questions prédéterminées (Ql) en fonction de la porte d'entrée sélectionnée, ladite séquence de questions sélectionnée formant ledit questionnaire.

4. Système (1) selon l'une des revendications 2 ou 3, dans lequel, pour au moins l'une desdites questions (Ql) de ladite séquence (Sij), le contrôleur (A) est agencé pour déterminer un score de réponse (Sl) en fonction de la réponse (Rl) apportée à ladite question, et pour générer ledit commentaire relatif à la consultation (Cp) en fonction d'au moins l'un desdits scores déterminés.

5. Système (1) selon la revendication précédente, dans lequel, pour chaque bloc de question (Bn), le contrôleur (A) est agencé pour générer un commentaire relatif à la consultation (Cp) en fonction d'au moins un score (Sl) déterminé en fonction d'une réponse (Rl) apportée à l'une des questions (Ql) dudit bloc.

6. Système (1) selon l'une des revendications 4 ou 5, dans lequel, pour au moins un bloc de questions (Bn), le contrôleur (A) est agencé pour afficher une partie seulement des questions (Ql) de ce bloc, dite préliminaire, et pour afficher le reste des questions de ce bloc en fonction d'au moins un score (Sl) déterminé en fonction d'une réponse (Rl) apportée à l'une des questions de ladite partie préliminaire.

7. Système (1) selon l'une des revendications précédentes, dans lequel, à l'issue du remplissage du questionnaire par le vétérinaire, le contrôleur (A) est agencé pour afficher, sur l'écran (31) du terminal mobile (3), l'ensemble des commentaires relatifs à la consultation (Cp) générés par le contrôleur à l'issue du remplissage du questionnaire, ainsi qu'un ensemble de composants graphiques de sélection (CB) chacun associé à l'une des commentaires affichés, le contrôleur étant agencé pour générer un rapport de consultation contenant les commentaires relatifs à la consultation sélectionnés au moyen desdits composants graphiques de sélection.

8. Système (1) selon l'une des revendications précédentes, **caractérisé en ce que** l'équipement connecté (2) est une paire de lunettes comportant un module de communication sans-fil (27), un système d'acquisition vidéo (25) et un écran (26), et **en ce qu'**il comporte une unité informatique (4) distante équipée d'un système d'acquisition et d'émission audio (41), d'un écran (42) et d'un module de communication sans-fil (44) apte à communiquer avec le module de communication sans-fil de l'équipement connecté.

9. Système selon la revendication précédente, dans lequel le module de communication sans-fil (27) de la paire de lunettes (2) est agencé pour transmettre les images acquises par le système d'acquisition vidéo (25) de la paire de lunettes au module de communication sans-fil (44) de l'unité informatique (4), l'unité informatique étant agencée pour afficher sur l'écran (42) de l'unité informatique les images reçues, dans lequel l'unité informatique comporte une interface (43) de modification d'au moins l'une desdites images reçues, et dans lequel l'unité informatique est agencée pour transmettre au module de communication sans-fil de de la paire de lunettes, via son module de communication sans-fil, une image modifiée au moyen de ladite interface de modification, la paire de lunettes étant agencée pour afficher ladite image modifiée sur l'écran (26) de la paire de lunettes.

10. Procédé de support à une consultation mis en œuvre par un terminal mobile (3) d'un système de consultation (1) selon l'une des revendications précédentes.

11. Programme d'ordinateur comprenant un code de programme qui est conçu pour mettre en œuvre le procédé selon la revendication précédente lorsque ledit programme est exécuté par un ordinateur.
